(19) **Europäisches Patentamt / European Patent Office / Office européen des brevets**

(11) **EP 4 778 911 A1**

(12) **EUROPEAN PATENT APPLICATION**
published in accordance with Art. 153(4) EPC

(43) Date of publication:
**22.07.2026 Bulletin 2026/30**

(21) Application number: **24880086.4**

(22) Date of filing: **15.10.2024**

(51) International Patent Classification (IPC):
$C07C\ 37/48$ $^{(2006.01)}$  $\quad$ $C07C\ 39/07$ $^{(2006.01)}$
$B01J\ 35/00$ $^{(2024.01)}$  $\quad$ $B01J\ 23/34$ $^{(2006.01)}$
$B01J\ 23/02$ $^{(2006.01)}$  $\quad$ $B01J\ 35/40$ $^{(2024.01)}$
$B01J\ 37/00$ $^{(2006.01)}$  $\quad$ $B01J\ 37/04$ $^{(2006.01)}$

(52) Cooperative Patent Classification (CPC):
**B01J 23/02; B01J 23/34; B01J 35/00; B01J 35/40; B01J 37/00; B01J 37/04; C07C 37/48; C07C 39/07**

(86) International application number:
**PCT/KR2024/015610**

(87) International publication number:
**WO 2025/084749 (24.04.2025 Gazette 2025/17)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: 16.10.2023 KR 20230137833
16.10.2023 KR 20230137834

(71) Applicant: Hanwha Solutions Corporation
Jung-gu
Seoul 04541 (KR)

(72) Inventors:
• **SEO, Hyun**
**Daejeon 34128 (KR)**
• **DO, Seung Hoe**
**Daejeon 34128 (KR)**
• **KIM, Youngjin**
**Daejeon 34128 (KR)**
• **KIM, Jiyeon**
**Daejeon 34128 (KR)**
• **SIM, Jaehun**
**Daejeon 34128 (KR)**

(74) Representative: **Berggren Oy**
**P.O. Box 16**
**Fabianinkatu 21**
**00101 Helsinki (FI)**

(54) **PREPARATION METHOD OF 2,6-DIMETHYLPHENOL**

(57)    The present invention relates to a method for preparing 2,6-dimethylphenol, and more particularly, to a method for preparing 2,6-dimethylphenol from o-cresol using a catalyst comprising manganese oxide.

EP 4 778 911 A1

**Description**

[Technical Field]

Cross-reference to Related Application(s)

This application claims the benefit of priority based on Korean Patent Application No. 10-2023-0137833 filed on October 16, 2023, and Korean Patent Application No. 10-2023-0137834 filed on October 16, 2023, the entire contents of which are incorporated herein by reference.

The present invention relates to a method for preparing 2,6-dimethylphenol, and more particularly, to a method for preparing 2,6-dimethylphenol using a catalyst comprising manganese oxide.

[Background Art]

Polyphenylene ether (PPE) is an engineering plastic material having excellent electrical properties, heat resistance, dimensional stability, low water absorption, and creep properties at high temperatures, and is widely used for electrical/electronic components and automobile parts requiring precise dimensions, and particularly, recently, various research and development efforts have been attempted by replacing automobile exterior panels made of steel to achieve weight reduction and energy reduction.

2,6-dimethylphenol is mainly used as a polymerization raw material for the production of polyphenylene ether (PPE), and is also widely used as an antioxidant, stabilizer, preservative, and antibacterial agent in various industrial fields. Meanwhile, impurities contained in 2,6-dimethylphenol inhibit the activity of a polymerization catalyst or deteriorate the quality of the produced polyphenylene ether during the polymerization of polyphenylene ether. Accordingly, in order to use 2,6-dimethylphenol as a polymerization raw material, high purity of 2,6-dimethylphenol is required.

2,6-dimethylphenol is mainly prepared by a chemical synthesis method. In conventional methods for preparing 2,6-dimethylphenol, toluene or m-cresol was used as a starting material, and the preparation was carried out through an alkylation reaction in the presence of a metal chloride catalyst. However, these methods have problems in that toxic substances may be generated during the manufacturing process, which may cause environmental pollution, strict control of reaction conditions is required, and productivity is low due to a long reaction time.

In addition, a method for obtaining 2,6-dimethylphenol through a methylation reaction of phenol in the presence of a solid catalyst has been proposed. However, the methylation reaction of phenol is a series reaction, which produces alkylphenol substances (o-cresol, 2,6-dimethylphenol, and trimethylphenol), making it difficult to selectively obtain 2,6-dimethylphenol, which is a target product, with high purity. In addition, trimethylphenol produced through over-methylation is difficult to reuse for reconversion to dimethylphenol, and thus has low practicality and is disposed of as waste, which adversely affects process economics. Furthermore, there is a problem in that polymerization of alkylphenol, which is a product, proceeds during the reaction process, causing polymer substances to carbonize on the catalyst, leading to deactivation of the catalyst.

Therefore, research is needed to develop a manufacturing method that can solve these problems and efficiently secure high selectivity of 2,6-dimethylphenol.

[Detailed Description of the Invention]

[Technical Problem]

An object of the present invention is to provide a preparation method having excellent reaction activity and high 2,6-dimethylphenol selectivity in the preparation of 2,6-dimethylphenol from o-cresol.

[Technical Solution]

In order to solve the above problems, the present specification provides a method for preparing 2,6-dimethylphenol, comprising the steps of: preparing a catalyst comprising manganese oxide represented by Chemical Formula 1 below; and a reaction step of methylating a reactant comprising o-cresol in the presence of the catalyst, wherein the catalyst is a manganese oxide single-component catalyst, or a composite catalyst comprising MgO and manganese oxide represented by Chemical Formula 1 below:

[Chemical Formula 1]     $Mn_xO_y$

x is an integer of 1 to 3, and

y is an integer of 1 to 4.

According to another example of the invention, in the preparing method, the manganese oxide represented by Chemical Formula 1 may comprise at least one selected from the group consisting of MnO, $MnO_2$, $Mn_2O_3$, and $Mn_3O_4$.

According to another example of the invention, in the preparing method, the manganese oxide single-component catalyst may be $Mn_3O_4$.

According to another example of the invention, in the preparing method, the composite catalyst may comprise MgO in an amount of 85 wt% or more.

According to another example of the invention, in the preparing method, when preparing the composite catalyst, the Mn content may be 0.5 to 15 wt% based on Mg.

According to another example of the invention, in the preparing method, the composite catalyst may be prepared by mechanically mixing MgO and manganese oxide by any one of the following methods a) to c):

a) A method of mixing MgO granules and manganese oxide in a granular state.
b) A method of mixing MgO and manganese oxide in a powder state and then preparing granules of the mixture.
c) A method of mixing MgO and manganese oxide into a slurry state in the presence of a solvent, followed by filtration and drying steps to prepare granules.

According to another example of the invention, in the preparing method, the composite catalyst may be prepared by doping Mn into MgO. In addition, a Mn precursor used for the Mn doping may be $Mn(NO_3)_2$.

According to another example of the invention, the reactant comprises o-cresol and methanol, and the molar ratio of the o-cresol:methanol may be 1:1 to 1:10. In addition, the reactant may further comprise water.

According to another example of the invention, the reaction step is performed in a fixed-bed reactor, and the reactant may be input to the reactor at 0.5 g/h to 5 g/h.

According to another example of the invention, the reaction step is performed in a fixed-bed reactor, and the space velocity (LHSV) of the reactant per the mass of the catalyst may be 0.1 $h^{-1}$ to 3 $h^{-1}$.

According to another example of the invention, in the preparing method, the reaction may proceed under a temperature condition of 350 to 450 °C.

According to another example of the invention, in the preparing method, the conversion of o-cresol may be 70% or more, and the selectivity of 2,6-dimethylphenol may be 85% or more.

The terms used herein are only used to describe exemplary embodiments and are not intended to limit the present invention.

Singular expressions include plural expressions unless the context clearly indicates otherwise.

In the present specification, terms such as "comprise," "include," or "have" are intended to describe implemented features, numbers, steps, components, or combinations thereof, and do not exclude the possibility of presence or addition of one or more other features, numbers, steps, components, combinations thereof.

Also, in the present specification, when a layer or an element is referred to as being formed "on" or "above" other layers or elements, it means that the layer or element is directly formed on the other layers or elements, or it means that another layer or element may be additionally formed between the layers, on an object, or on a substrate.

Since the present invention may be subjected to various modifications and may have various forms, specific embodiments will be exemplified and described in detail below. However, this is not intended to limit the present invention to a specific disclosure form, and it should be understood to include all modifications, equivalents, or substitutes included in the spirit and technical scope of the present invention.

Hereinafter, the present invention will be described in detail.

The present invention provides a method for preparing 2,6-dimethylphenol, comprising the steps of: preparing a catalyst comprising manganese oxide represented by Chemical Formula 1 below; and a reaction step of methylating a reactant comprising o-cresol in the presence of the catalyst, wherein the catalyst is a manganese oxide single-component catalyst, or a composite catalyst comprising MgO and manganese oxide represented by Chemical Formula 1 below:

[Chemical Formula 1]      $Mn_xO_y$

x is an integer of 1 to 3, and
y is an integer of 1 to 4.

In the conventional method for preparing 2,6-dimethylphenol through a methylation reaction of phenol, a magnesium oxide-based catalyst or an iron (Fe)-based catalyst has been mainly used as a solid catalyst. However, the magnesium oxide-based catalyst required a high-temperature reaction of about 450 °C or more for high activity, which led to a problem of a shortened catalyst lifetime. This problem, in turn, consequently caused a decrease in the selectivity of 2,6-dimethylphenol and also necessitated a catalyst reuse process, resulting in cost problems. In addition, the iron (Fe)-based catalyst, for example, an iron-vanadium-based catalyst, had a problem of being suitable only for a specific type of reactor such as a fluidized bed reactor due to frequent deactivation of the catalyst.

In addition, the process for preparing 2,6-dimethylphenol through alkylation of phenol has a problem that selectivity of 2,6-dimethylphenol is low because by-products such as o-cresol and 2,4,6-trimethylphenol are produced in addition to 2,6-dimethylphenol, which is a target product, due to a series reaction of phenol.

In order to prepare 2,6-dimethylphenol from o-cresol, a process of selectively methylating carbon 6 of o-cresol, which is a reactant, is required. At this time, a process of generating carbonium ions using a catalyst for methylation is performed, but conventionally known magnesium oxide (MgO)-based catalysts are base catalysts and lack strong acid sites, so the reaction is required to proceed at a high temperature for carbonium ion generation. If the reaction proceeds at such a high temperature, it may cause an increase in over-methylation and generation of by-products.

Accordingly, the inventors of the present invention confirmed that, when 2,6-dimethylphenol is prepared through a methylation reaction using a reactant comprising o-cresol as a starting material in the presence of a catalyst comprising manganese oxide, the binding energy of Mn, which is a metal component, and oxygen, which is a non-metal component, in the crystal structure of manganese oxide is relatively low, and the reaction of generating formaldehyde from methanol is promoted due to oxidation-reduction characteristics of manganese oxide included in the composite catalyst, so that the present reaction can proceed at a relatively low temperature. It was confirmed that the reaction is selectively promoted due to such oxidation-reduction characteristics of the catalyst of the present invention, and the present invention was completed by finding that high conversion of o-cresol and high selectivity of 2,6-dimethylphenol, which are reaction materials, can be simultaneously satisfied, and the lifetime of the catalyst can also be improved.

As described above, the catalyst used in the present invention is a catalyst comprising manganese oxide represented by Chemical Formula 1 below:

[Chemical Formula 1]          $Mn_xO_y$

x is an integer of 1 to 3, and
y is an integer of 1 to 4.

The manganese oxide may exist in various forms depending on the oxidation state of manganese. For example, the manganese oxide may exist in forms such as $MnO$, $MnO_2$, $Mn_2O_3$, $Mn_3O_4$, $Mn_2O_7$ and the like. Preferably, the manganese oxide represented by Chemical Formula 1 may comprise at least one selected from the group consisting of $MnO$, $MnO_2$, $Mn_2O_3$, and $Mn_3O_4$, but is not limited thereto.

The catalyst comprising manganese oxide of the present invention is a manganese oxide single-component catalyst, or a composite catalyst comprising MgO and manganese oxide represented by Chemical Formula 1 below. As used herein, 'single component' means that the various forms of manganese oxide described above exist only in any one form in the catalyst.

According to an embodiment of the present invention, the manganese oxide single-component catalyst may be $Mn_3O_4$.

Also, in the present specification, "composite catalyst" means a single structure in which each component of the catalyst is physically and/or chemically mixed and/or bonded, and the forms of MgO and manganese oxide in the structure are not particularly limited. That is, the MgO and manganese oxide composite catalyst may be a form in which MgO and manganese oxide are physically mixed, a form in which MgO and manganese oxide are chemically bonded, or a form in which, within the composite catalyst, a portion of the MgO and manganese oxide is physically mixed and another portion is chemically bonded.

According to an embodiment of the present invention, the composite catalyst may comprise MgO in an amount of 85 wt% or more. MgO is a main component of the composite catalyst used in the present invention, and it is preferable in that the reaction activity may be increased when MgO is included in an amount of 85 wt% or more in the composite catalyst as described above. If the amount of MgO is less than 85 wt%, a problem of an increase in by-products due to an over-alkylation reaction may occur. Meanwhile, the upper limit of the MgO content in the composite catalyst is not particularly limited, but for example, the composite catalyst may comprise MgO in an amount of 99.99 wt% or less, or 99.5 wt% or less, or 99.2 wt% or less, or 99.1 wt% or less. If the upper limit of the MgO content exceeds the above range, it may be difficult to achieve high conversion of the reactant. Preferably, the composite catalyst may comprise MgO in an amount of 87 wt% or more, 89 wt% or more, or 90 wt% or more, and 99.99 wt% or less, 99.5 wt% or less, 99.2 wt% or less, or 99.1 wt% or less.

According to an embodiment of the present invention, when preparing the composite catalyst, the Mn content may be 0.5 to 15 wt% based on Mg. When a catalyst prepared by using the Mn content less than 0.5 wt% based on Mg, a reaction is required under high-temperature conditions to obtain high conversion of the reactant, which is not preferable, and when a catalyst prepared by using the Mn content more than 15 wt% based on Mg, selectivity of 2,6-dimethylphenol may be lowered due to a side reaction. Preferably, when preparing the composite catalyst, the Mn content may be 0.7 to 14 wt%, or 0.8 to 13 wt%, or 0.9 to 12 wt%, or 1.0 to 11.5 wt% based on Mg.

Meanwhile, a method for preparing the composite catalyst is not particularly limited, but as described later, it may be prepared by mechanically mixing of MgO and manganese oxide, or by doping Mn into MgO.

For example, although not particularly limited, mechanically mixing of each oxide may be performed by any one of the following methods a) to c):

a) A method of mixing MgO granules and manganese oxide in a granular state.
b) A method of mixing MgO and manganese oxide in a powder state and then preparing granules of the mixture.
c) A method of mixing MgO and manganese oxide into a slurry state in the presence of a solvent, followed by filtration and drying steps to prepare granules.

Specifically, according to the method a), after preparing MgO granules and manganese oxide granules, the granules of each oxide may be mixed as they are using a stirrer or the like. At this time, for proper mixing of the granules, a non-reactive solvent such as water or the like may be used as needed, and equipment for mixing each oxide is not particularly limited.

According to an embodiment of the present invention, the granule size of the MgO granule and manganese oxide granule may be 650 $\mu$m or less. When the composite catalyst is prepared using a granule size in the above range, it is preferable to reduce internal diffusion resistance of the catalyst, and for example, granules having a size of 50 $\mu$m to 550 $\mu$m, or 100 $\mu$m to 500 $\mu$m may be used. Meanwhile, the granule size means the particle diameter.

In addition, according to method b), MgO and manganese oxide may be mixed by a method that involves mixing the raw materials themselves in powder form and subsequently preparing granules of the mixture. At this time, a mixing method for each oxide powder and a preparation method for the granules may apply methods generally used in the art, and a non-reactive solvent such as water may also be used as needed, similar to a) above.

In addition, as an example, the method c) may be a method of adding MgO to a solvent to form a slurry, adding manganese oxide to the slurry to prepare a mixed slurry, filtering and drying them to remove the solvent, and then preparing granules. Herein, the mixing time, the filtration method, and the drying method, among others, for preparing the mixed slurry may be adopted from methods generally used in the art. In addition, a solvent for slurry preparation is not particularly limited, but preferably water may be used.

Meanwhile, according to an embodiment of the present invention, the composite catalyst may be prepared by doping Mn into MgO. Methods for doping Mn into MgO may include a precipitation method, a coprecipitation method, an impregnation method, and the like, but are not limited thereto. In addition, a Mn precursor solution may be used to dope Mn. Usable Mn precursors are not particularly limited, but may be Mn-containing acetate, nitrate, sulfate, halide, sulfide, hydroxide, oxide, oxyhydroxide, or a combination thereof. Specifically, manganese oxides themselves such as $Mn_2O_3$, $MnO_2$, $Mn_3O_4$, and the like; manganese salts such as $MnCO_3$, $Mn(NO_3)_2$, $MnSO_4$, manganese acetate, manganese dicarboxylate, manganese citrate, and manganese fatty acid salts; manganese oxyhydroxide; manganese chloride; or a combination thereof may be used, but are not limited thereto. Preferably, $Mn(NO_3)_2$ may be used.

Specifically, the method of doping Mn into MgO may be performed by an incipient wetness impregnation method. First, after measuring a pore volume of MgO, a Mn precursor solution corresponding to the pore volume of MgO is prepared. At this time, Mn concentration in the Mn precursor solution may be determined in consideration of the ratio of Mg/Mn in the composite catalyst described above. As a solvent for the Mn precursor solution, water or alcohol may be used. When doping Mn according to the incipient wetness impregnation method, in order to achieve a desired Mg/Mn ratio, impregnation may be repeated several times within a limited pore volume, and a drying step may be added as needed for each impregnation. When impregnation is completed, a composite catalyst is prepared through drying and calcination steps, and then may be prepared in the form of a granule.

When the manganese oxide catalyst represented by Chemical Formula 1 of the present invention described above is applied to a 2,6-dimethylphenol conversion reaction through methylation of o-cresol, it exhibits excellent conversion of o-cresol and, simultaneously, by selectively substituting methyl at the 2-position of o-cresol while blocking an over-methylation reaction, it can improve the selectivity of 2,6-dimethylphenol.

In the present invention, o-cresol, which is a reactant, is generally produced as an intermediate product in a reaction for generating 2,6-dimethylphenol from phenol. At this time, the conventional 2,6-dimethylphenol generation reaction through a continuous methylation reaction of phenol is accompanied by over-methylation products such as trimethylphenol in addition to o-cresol which is an intermediate product. In addition, various by-products such as methylanisole, dimethylanisole, 2,3-dimethylphenol, 2,5-dimethylphenol, 3,4-dimethylphenol, and trimethylphenol may be generated in the methylation reaction of o-cresol as well, depending on the substitution position and degree of methyl group substitution.

It was founded that the catalyst comprising the manganese oxide represented by Chemical Formula 1, applied in the above-described present invention, selectively proceeds with a one-step methylation reaction of o-cresol. Therefore, when the methylation reaction of o-cresol is performed using the catalyst, generation of intermediate products and over-methylation products such as trimethylphenol can be suppressed.

Meanwhile, in the present invention, the reactant may comprise a methylating agent in addition to o-cresol. The methylating agent is not particularly limited, but methanol may be used.

According to an embodiment of the present invention, the reactant comprises o-cresol and methanol, and the molar ratio of o-cresol:methanol may be 1:1 to 1:10. When the molar ratio of o-cresol:methanol is less than 1:1, it is difficult to achieve a high conversion rate, and when it exceeds 1:10, there may be a problem in that a significant amount of methanol is decomposed to induce a side reaction, leading to generation of by-products. Preferably, the molar ratio of o-cresol:methanol may be 1:1.5 or more, 1:2 or more, 1:2.5 or more, or 1:3 or more, and 1:9 or less, 1:8 or less, 1:7 or less, 1:6 or less, or 1:5 or less.

In addition, in the preparation method of the present invention, although not an essential component, the reactant may further comprise water in addition to o-cresol and the methylating agent. In the methylation reaction of o-cresol, water is input together with the other reactants to prevent coking of the catalyst surface, and thereby may maintain catalyst activity even during prolonged reactions, and may suppress a methanol decomposition reaction when methanol is used as a methylating agent.

According to an embodiment of the present invention, a molar ratio of o-cresol:water may be 1:0.1 to 1:2. Preferably, when water is included as a reactant, the molar ratio of o-cresol:water may be 1:0.5 to 1:1.5, 1:0.7 to 1:1.3, or 1:0.9 to 1:1.

Meanwhile, a reactor applicable to the methylation reaction of o-cresol of the present invention is not particularly limited. That is, the catalyst of the present invention has a low catalyst deactivation degree over time due to carbonization or the like as compared with a conventional catalyst comprising iron, and thus may be applied to both fixed-bed reactors and fluidized-bed reactors, and the like.

According to an embodiment of the present invention, the reaction step is performed in a fixed-bed reactor, and the reactant may be input to the reactor at 0.5 g/h to 5 g/h. When the reactant is input in the above range, a contact time with the catalyst may be appropriately maintained. Preferably, the flow rate of methanol in the reactant may be input at 0.7 g/h or more, 1.0 g/h or more, 1.5 g/h or more, or 1.7 g/h or more, and 4 g/h or less, 3.5 g/h or less, or 3 g/h or less.

According to an embodiment of the present invention, the reaction step is performed in a fixed-bed reactor, and the space velocity (LHSV) of the reactant per the mass of catalyst may be $0.1 \ h^{-1}$ to $3 \ h^{-1}$. When the space velocity of methanol per mass of catalyst is within the above range, the contact time with the catalyst may be appropriately maintained. Preferably, the space velocity may be input at $0.5 \ h^{-1}$ or more, $0.7 \ h^{-1}$ or more, or $1.0 \ h^{-1}$ or more, and $2.7 \ h^{-1}$ or less, $2.5 \ h^{-1}$ or less, or $2.0 \ h^{-1}$ or less.

According to an embodiment of the present invention, the reaction may proceed under a temperature condition of 350 °C to 450 °C. Through the reaction of methylating o-cresol at a temperature of 450 °C or less as in one embodiment, the lifetime of the catalyst may be improved, and generation of side reactions may be suppressed. Preferably, it may proceed under temperature conditions of 370 °C or higher, 400 °C or higher, or 420 °C or higher, and 445 °C or lower, or 440 °C or lower.

According to an embodiment of the present invention, the conversion of o-cresol may be 70% or more, and the selectivity of 2,6-dimethylphenol may be 85% or more. Preferably, the conversion of o-cresol may be 72% or more, 75% or more, or 77% or more, and may be 99% or less, 95% or less, 93% or less, or 91% or less. Also, preferably, the selectivity of 2,6-dimethylphenol may be 86% or more, 88% or more, 90% or more, or 92% or more, and may be 99% or less, 98% or less, or 97% or less.

In addition, when the single-component catalyst is applied, the selectivity of 2,6-dimethylphenol may be 90% or more. Preferably, when the single-component catalyst is applied, the selectivity of 2,6-dimethylphenol may be 92% or more, 93% or more, or 94% or more.

As described above, when the catalyst comprising manganese oxide of the present invention is applied to a methylation reaction of a reactant comprising o-cresol, productivity of 2,6-dimethylphenol can be increased.

[Effects of the Invention]

As described above, the method for preparing 2,6-dimethylphenol from o-cresol in the presence of a catalyst comprising manganese oxide according to the present invention has excellent selectivity for 2,6-dimethylphenol and also has excellent catalyst stability, so that it may be used for a long time.

[Mode for Carrying Out the Invention]

Hereinafter, preferred embodiments are presented to facilitate understanding of the invention. However, the following embodiments are only for illustrating the present invention, and the present invention is not limited thereto.

**<Preparation Example>**

**Preparation Example 1**

Particles of MgO and of MnO$_2$ samples (a manganese oxide) were pelletized at the same pressure using a hydraulic press, followed by grinding and sieving processes to prepare particles with a size of 212 to 425 $\mu$m. The prepared MgO (10 g) and manganese oxide (1.0603 g) were physically mixed in a granule state to prepare a composite catalyst (Mn/Mg(w/w) = 11.11 %).

**Preparation Example 2**

MgO (10 g) and a sample of manganese oxide, MnO$_2$ (1.0603 g), were first mixed in an unprocessed powder state, and then processed into granules with a size of 212 to 425 $\mu$m through pelletizing using a hydraulic press, followed by grinding and sieving processes, to prepare a composite catalyst.

**Preparation Example 3**

MgO (10 g) and MnO$_2$ (1.0603 g) as manganese oxide were dispersed in an excess of water (30 cc) to make a slurry state, and then stirred at 30 °C for 3 hours. Thereafter, a mixture was prepared through filtration and drying steps (performed at 70 °C for 24 hours in a vacuum oven). Then, it was processed into granules with a size of 212 to 425 $\mu$m through pelletizing using a hydraulic press, followed by grinding and sieving processes, to prepare a composite catalyst.

**Preparation Example 4**

A composite catalyst was prepared in the same manner as in Preparation Example 2, except that MgO (10 g) was used and MnO$_2$ (0.0964 g) was used as manganese oxide (Mn/Mg(w/w) = 1.01%).

**Preparation Example 5**

The pore volume value of MgO was measured (0.72 cc/g) through pore property analysis (Surface Area Analyzer (ASAP2420), pretreatment at 100 °C for 3h).
Doping was performed on 10 g of MgO by slowly adding a Mn(NO3)$_2$ aqueous solution (7.2 cc) corresponding to the pore volume of 10 g of MgO. At this time, 0.1984 g of Mn(NO$_3$)$_2$ was used such that Mn was about 1 wt% of Mg. Thereafter, drying (overnight, 70 °C) and calcination (1h, same temperature as the reaction temperature) steps were performed, and the prepared powder was pelletized using a hydraulic press, then subjected to grinding and sieving processes, to produce 212 to 425 $\mu$m-sized granules, thereby preparing a composite catalyst.

**<Embodiments>**

**Example 1-1**

1 g of MnO (Sigma-Aldrich) catalyst was charged into a tubular reactor having a diameter of 1/2 inch and a height of 50 cm. O-cresol, methanol, and water were mixed in a molar ratio of 1:4:1, and the resulting mixture was preheated to 250 °C through a preheater prior to being introduced into the tubular reactor, and then injected into the reactor at a rate of 2 g/h. Thereafter, the reaction was carried out for 5 hours while maintaining the reactor temperature and pressure at 440 °C and atmospheric pressure, thereby preparing 2,6-dimethylphenol (2,6-DMP).

**Example 1-2**

2,6-DMP was prepared in the same manner as in Example 1, except that MnO$_2$ (Sigma-Aldrich) catalyst was used instead of the catalyst prepared in Preparation Example 1.

**Example 1-3**

2,6-DMP was prepared in the same manner as in Example 1, except that Mn$_3$O$_4$ (SAMCHUN CHEMICALS) catalyst was used instead of the catalyst prepared in Preparation Example 1.

**Comparative Example 1-1**

2,6-DMP was prepared in the same manner as in Example 1, except that the MgO (manufactured by Kyowa Chemical Industry, Kyowamag 150) catalyst was used instead of the catalyst prepared in Preparation Example 1.

**Comparative Example 1-2**

2,6-DMP was prepared in the same manner as in Example 1-1, except that $CeO_2$ (Sigma-Aldrich) catalyst was used instead of the catalyst prepared in Preparation Example 1.

**Example 2-1**

2 g of the catalyst prepared in Preparation Example 3 was charged into a tubular reactor having a diameter of 1/2 inch and a height of 50 cm. O-cresol, methanol, and water were mixed in a molar ratio of 1:4:1. The mixture was preheated to 250 °C through a preheater before being input into the tubular reactor, and then injected into the reactor at a rate of 2 g/h (LHSV: 1 $h^{-1}$). Thereafter, the reaction was carried out for 5 hours while maintaining the reactor temperature and pressure at 420 °C and atmospheric pressure, thereby preparing 2,6-dimethylphenol (2,6-DMP).

**Examples 2-2 to 2-9**

2,6-dimethylphenol (2,6-DMP) was prepared in the same manner as in Example 2-1, except that the reaction temperature and LHSV were changed as described in Table 2 below.

**Example 3-1**

1 g of the catalyst prepared in Preparation Example 1 was charged into a tubular reactor having a diameter of 1/2 inch and a height of 50 cm. O-cresol, methanol, and water were mixed in a molar ratio of 1:4:1, and this mixture was preheated to 250 °C through a preheater before being input into the tubular reactor, and then injected into the reactor at a rate of 2 g/h. Thereafter, the reaction was carried out for 5 hours while maintaining the reactor temperature and pressure at 440 °C and atmospheric pressure, thereby preparing 2,6-dimethylphenol (2,6-DMP).

**Example 3-2**

2,6-DMP was prepared in the same manner as in Example 1, except that the catalyst prepared in Preparation Example 2 was used instead of the catalyst prepared in Preparation Example 1.

**Example 3-3**

2,6-DMP was prepared in the same manner as in Example 1, except that the catalyst prepared in Preparation Example 3 was used instead of the catalyst prepared in Preparation Example 1.

**Example 3-4**

2,6-DMP was prepared in the same manner as in Example 1, except that the catalyst prepared in Preparation Example 4 was used instead of the catalyst prepared in Preparation Example 1.

**Example 3-5**

2,6-DMP was prepared in the same manner as in Example 1, except that the catalyst prepared in Preparation Example 5 was used instead of the catalyst prepared in Preparation Example 1.

**Comparative Example 3-1**

2,6-DMP was prepared in the same manner as in Example 1, except that MgO (manufactured by Kyowa Chemical Industry, Kyowamag 150) catalyst was used instead of the catalyst prepared in Preparation Example 1.

**<Experimental Example>**

(1) Conversion and Selectivity

Analytical method: gas chromatography analysis, use of an HP-5 column conversion (%) = ((the number of moles of o-cresol reacted)/(the number of moles of o-cresol input)) × 100 selectivity (%) = ((the number of moles of 2,6-DMP produced)/(total number of moles of products)) × 100

The reaction activity results of Examples 1-1 to 1-3 and Comparative Examples 1-1 and 1-2 are shown in Table 1 below. In addition, the reaction activity results of Examples 2-1 to 2-9 are shown in Table 2 below. In addition, the reaction activity results of Examples 3-1 to 3-5 and Comparative Example 3-1 are shown in Table 3 below.

[Table 1]

| | o-cresol conversion (%) | 2,6-DMP selectivity (%) | 2,4,6-TMP selectivity | 2,4-DMP | DMPs | Anisole |
|---|---|---|---|---|---|---|
| Example 1-1 | 3.3 | 100 | 0 | 0 | 0 | 0 |
| Example 1-2 | 49.8 | 99.6 | 0.1 | 0 | 0.1 | 0 |
| Example 1-3 | 85.8 | 98.7 | 0.7 | 0 | 0.4 | 0 |
| Comparative Example 1-1 | 68.6 | 85.6 | 8.6 | 3.5 | 0.4 | 1.1 |
| Comparative Example 1-2 | 10.7 | 86.9 | 0.4 | 1.7 | 4.0 | 1.6 |

As can be seen from Table 1, in the case of Examples 1-1 to 1-3 in which 2,6-DMP was prepared using the manganese oxide catalyst of the present invention, it was confirmed that the selectivity of 2,6-DMP, which is a target product, was higher than that of Comparative Examples using a MgO single-component catalyst and a CeO2 single-component catalyst.

[Table 2]

| | Feed molar ratio (o-cresol:methanol:water) | reaction temperature (°C) | LHSV (h-1) | o-cresol conversion (%) | 2,6-DMP selectivity (%) | 2,6-DMP yield (%) |
|---|---|---|---|---|---|---|
| Example 2-1 | 1:4:1 | 420 | 1 | 97.7 | 98.1 | 95.8 |
| Example 2-2 | 1:4:1 | 430 | 1.5 | 94.9 | 98.5 | 93.4 |
| Example 2-3 | 1:4:1 | 440 | 1.5 | 97.2 | 98.3 | 95.6 |
| Example 2-4 | 1:5:1 | 400 | 1 | 97.7 | 98.3 | 96.0 |
| Example 2-5 | 1:6:1 | 400 | 1 | 99.9 | 97.2 | 97.2 |
| Example 2-6 | 1:6:1 | 440 | 1.5 | 99.1 | 96.9 | 96 |
| Example 2-7 | 1:6:1 | 430 | 1.5 | 98.3 | 97.3 | 95.6 |
| Example 2-8 | 1:6:1 | 420 | 1.5 | 96.3 | 98 | 94.4 |
| Example 2-9 | 1:6:1 | 420 | 2 | 91.8 | 98.5 | 90.4 |
| Example 2-10 | 1:6:0 | 380 | 1 | 95.4 | 98.7 | 94.2 |
| Example 2-11 | 1:6:0 | 400 | 1 | 99.9 | 97.4 | 97.3 |
| Example 2-12 | 1:5:0 | 400 | 1 | 99.8 | 98 | 97.7 |

As can be seen from Table 2, in Examples 2-1 to 2-12 in which the manganese oxide single-component catalyst of the present invention was used, it was confirmed that the yield of the conversion reaction of 2,6-DMP from o-cresol was at a high level of 90% or more.

[Table 3]

| | Mn/Mg(w/w) | o-cresol conversion (%) | 2,6-DMP selectivity (%) | 2,4,6-TMP selectivity | DMP Isomer | Anisole |
|---|---|---|---|---|---|---|
| Example 3-1 | 10 | 90.3 | 85.8 | 12.0 | 1.1 | 0.6 |
| Example 3-2 | 10 | 77.4 | 92.1 | 5.5 | 1.6 | 0.4 |
| Example 3-3 | 10 | 89.8 | 80.8 | 16.0 | 1.9 | 0.9 |
| Example 3-4 | 1 | 80.1 | 96.1 | 1.9 | 1.4 | 0.5 |
| Example 3-5 | 1 | 70.5 | 93.3 | 5.2 | 1.2 | 0.2 |
| Comparative Example 3-1 | - | 68.6 | 85.6 | 8.6 | 3.8 | 1.1 |

As can be seen from Table 3, in the case of Examples 3-1 to 3-5 in which 2,6-DMP was prepared using the Mg and Mn composite catalyst of the present invention, it was confirmed that, compared to the Comparative Example using a MgO single-component catalyst, both the conversion of o-cresol was higher and the selectivity of 2,6-DMP, which is a target product, was also high.

**Claims**

1.  A method for preparing 2,6-dimethylphenol, comprising the steps of: preparing a catalyst comprising manganese oxide represented by Chemical Formula 1 below; and a reaction step of methylating a reactant comprising o-cresol in the presence of the catalyst, wherein the catalyst is a manganese oxide single-component catalyst, or a composite catalyst comprising MgO and manganese oxide represented by Chemical Formula 1 below:

    [Chemical Formula 1]       $Mn_xO_y$

    x is an integer of 1 to 3, and y is an integer of 1 to 4.

2.  The method for preparing 2,6-dimethylphenol according to claim 1, wherein the manganese oxide represented by Chemical Formula 1 comprises at least one selected from the group consisting of $MnO$, $MnO_2$, $Mn_2O_3$, and $Mn_3O_4$.

3.  The method for preparing 2,6-dimethylphenol according to claim 1, wherein the manganese oxide single-component catalyst is $Mn_3O_4$.

4.  The method for preparing 2,6-dimethylphenol according to claim 1, wherein the composite catalyst comprises MgO in an amount of 85 wt% or more.

5.  The method for preparing 2,6-dimethylphenol according to claim 1, wherein, when preparing the composite catalyst, Mn content is 0.5 to 15 wt% based on Mg.

6.  The method for preparing 2,6-dimethylphenol according to claim 1, wherein the composite catalyst is prepared by mechanically mixing MgO and manganese oxide by any one of the following methods a) to c):

    a) A method of mixing MgO granules and manganese oxide in a granular state.
    b) A method of mixing MgO and manganese oxide in a powder state and then preparing granules of the mixture.
    c) A method of mixing MgO and manganese oxide into a slurry state in the presence of a solvent, followed by filtration and drying steps to prepare granules.

7.  The method for preparing 2,6-dimethylphenol according to claim 1, wherein the composite catalyst is prepared by doping Mn into MgO.

8.  The method for preparing 2,6-dimethylphenol according to claim 7, for producing 2,6-dimethylphenol, wherein a Mn precursor used for the Mn doping is $Mn(NO_3)_2$.

9. The method for preparing 2,6-dimethylphenol according to claim 1, wherein the reactant comprises o-cresol and methanol, and the molar ratio of the o-cresol:methanol is 1:1 to 1:10, for preparing 2,6-dimethylphenol.

10. The method for preparing 2,6-dimethylphenol according to claim 9, wherein the reactant further comprises water, and the molar ratio of the o-cresol:water is 1:0.1 to 1:2.

11. The method for preparing 2,6-dimethylphenol according to claim 1, wherein the reaction step is performed in a fixed-bed reactor, and the reactant is input to the reactor at 0.5 g/h to 5 g/h.

12. The method for preparing 2,6-dimethylphenol according to claim 1, wherein the reaction step is performed in a fixed-bed reactor, and the space velocity (LHSV) of the reactant per the mass of catalyst is 0.1 $h^{-1}$ to 3 $h^{-1}$.

13. The method for preparing 2,6-dimethylphenol according to claim 1, wherein the reaction proceeds under a temperature condition of 350 to 450 °C.

14. The method for preparing 2,6-dimethylphenol according to claim 1, wherein the conversion of o-cresol is 70% or more, and the selectivity of 2,6-dimethylphenol is 85% or more.

## INTERNATIONAL SEARCH REPORT

| International application No. |
|---|
| **PCT/KR2024/015610** |

| A. | CLASSIFICATION OF SUBJECT MATTER |
|---|---|

**C07C 37/48**(2006.01)i; **C07C 39/07**(2006.01)i; **B01J 35/00**(2006.01)i; **B01J 23/34**(2006.01)i; **B01J 23/02**(2006.01)i; **B01J 35/40**(2024.01)i; **B01J 37/00**(2006.01)i; **B01J 37/04**(2006.01)i

According to International Patent Classification (IPC) or to both national classification and IPC

| B. | FIELDS SEARCHED |
|---|---|

Minimum documentation searched (classification system followed by classification symbols)

C07C 37/48(2006.01); B01J 23/22(2006.01); B01J 23/745(2006.01); B01J 23/847(2006.01); B01J 23/889(2006.01); C07C 37/11(2006.01); C07C 37/68(2006.01); C07C 39/06(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 2,6-디메틸페놀(2,6-dimethylphenol), o-크레졸(o-cresol), 망간 산화물(manganese oxide), 촉매(catalyst)

| C. | DOCUMENTS CONSIDERED TO BE RELEVANT |
|---|---|

| Category* | Citation of document, with indication, where appropriate, of the relevant passages | Relevant to claim No. |
|---|---|---|
| Y<br>A | LI, K.-T. et al. Methylation of phenol to 2,6-dimethylphenol on a manganese oxide catalyst. Ind. Eng. Chem. Res. 1993, vol. 32, pp. 1007-1011.<br>See abstract; Experimental and Reaction Kinetics sections; and tables 1 and 2. | 1-6,9-14<br>7,8 |
| Y | KR 10-1067656 B1 (KOREA RESEARCH INSTITUTE OF CHEMICAL TECHNOLOGY) 27 September 2011 (2011-09-27)<br>See paragraphs [0001] and [0006]; and examples 3 and 4. | 1-6,9-14 |
| A | KR 10-1070169 B1 (KOREA RESEARCH INSTITUTE OF CHEMICAL TECHNOLOGY) 05 October 2011 (2011-10-05)<br>See claims 1-4, 7 and 8-12. | 1-14 |
| A | KR 10-2013-0110931 A (KOREA RESEARCH INSTITUTE OF CHEMICAL TECHNOLOGY) 10 October 2013 (2013-10-10)<br>See claim 1; and paragraphs [0036] and [0037]. | 1-14 |

☑ Further documents are listed in the continuation of Box C.  ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **04 February 2025** | **04 February 2025** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

| International application No. |
|---|
| **PCT/KR2024/015610** |

### C. DOCUMENTS CONSIDERED TO BE RELEVANT

| Category* | Citation of document, with indication, where appropriate, of the relevant passages | Relevant to claim No. |
|---|---|---|
| A | KR 10-2010-0046970 A (KOREA RESEARCH INSTITUTE OF CHEMICAL TECHNOLOGY) 07 May 2010 (2010-05-07)<br>See claims 1, 6, 9, 10 and 13. | 1-14 |

Form PCT/ISA/210 (second sheet) (July 2022)

**INTERNATIONAL SEARCH REPORT**
Information on patent family members

International application No.

**PCT/KR2024/015610**

| Patent document cited in search report | | | Publication date (day/month/year) | Patent family member(s) | | | Publication date (day/month/year) |
|---|---|---|---|---|---|---|---|
| KR | 10-1067656 | B1 | 27 September 2011 | KR  10-2011-0007827 | A | | 25 January 2011 |
| KR | 10-1070169 | B1 | 05 October 2011 | KR  10-2011-0107628 | A | | 04 October 2011 |
| KR | 10-2013-0110931 | A | 10 October 2013 | KR | 10-1357113 | B1 | 11 February 2014 |
| KR | 10-2010-0046970 | A | 07 May 2010 | KR | 10-0964800 | B1 | 22 June 2010 |

Form PCT/ISA/210 (patent family annex) (July 2022)

**EP 4 778 911 A1**

**Patent documents cited in the description**

- KR 1020230137833
- KR 1020230137834